# EUROPEAN PATENT SPECIFICATION

(11) **EP 3 231 779 B1**
(45) Date of publication and mention of the grant of the patent: **14.11.2018**
(21) Application number: 16165521.2
(22) Date of filing: 15.04.2016
(51) Int. Cl.: C04B 2/10, C04B 7/44

(54) **PROCESS AND PLANT FOR MANUFACTURING CEMENT IN THE OXYFUEL MODE**
VERFAHREN UND ANLAGE ZUR HERSTELLUNG VON ZEMENT IM OXYFUEL-MODUS
PROCÉDÉ ET INSTALLATION DE FABRICATION DE CIMENT DANS LE MODE D'OXYCOMBUSTION

(43) Date of publication of application: 18.10.2017
(73) Proprietor: HeidelbergCement AG, 69120 Heidelberg (DE)
(72) Inventor: THEULEN, Jan, 6061 CE Posterholt (NL)
(74) Representative: Zellentin & Partner mbB Patentanwälte

(56) References cited:
- WO-A1-2009/036095
- WO-A1-2015/055349
- DE-A1-102008 059 370
- DE-A1-102010 019 330

## Description

The present invention relates to a process and plant for manufacturing cement, especially in the oxyfuel mode, and to a use of the oxygen generated by photoautotrophic organisms as oxygen feed for burners in a cement plant. The application further relates to the analogous process and plant for making lime.

The cement industry is (under the umbrella of ECRA, European Cement Research Academy) working on a so-called oxyfuel cement kiln, see e.g.: http://www.ecra-online.org/fileadmin/redaktion/files/pdf/ ECRA_Technical_Report_CCS_Phase_III.pdf, http://www.worldcement.com/europe-cis/10042015/ECRA-research-on-carbon-capture-in-cement-industry-661/ and http://www.worldcement.com/europe-cis/10042015/ECRA-research-on-carbon-capture-in-cement-industry-662/.

An oxyfuel kiln is very similar to a traditional cement or lime kiln, however it is operated differently. The exhaust gas is being recirculated to the cooler inlet, to replace ambient air. In order to have enough oxygen at the flame of the kiln (> 20%), pure oxygen (or at least air that has been stripped of its nitrogen) is injected at the cooler and/or kiln inlet. The amount of oxygen needed is so high that an oxyfuel kiln of usual commercial scale needs an on-site oxygen generating unit that requires a large amount of electrical energy.

As a result of the recycling of the exhaust gas the CO₂-concentration is lifted from 25% for regular kilns to >70% for oxyfuel operation mode. After enrichment of the exhaust gas, finally a part of the exhaust gas has to be vented to allow new oxygen to enter the system.

The CO₂ level of the exhaust gas can either be further concentrated (by capturing techniques) or the exhaust gas can be liquefied as it is for further steps (storage and/or utilization). The capture of high concentrated CO₂ when running in oxyfuel-mode is considered to be the most economical solution (better than the end-of-pipe techniques like amine scrubbers for kilns in regular operation mode with ambient air). Still it is very expensive due to the high additional costs of operation.

It has already been suggested to use CO₂ from exhaust gas as feed for photoautothrophic organisms that generate valuable products from it when exposed to light. Such organisms typically build-up biomass that is subsequently converted to the desired product. In an improved more recent approach organisms have been obtained by genetic modification that can convert CO₂ directly into carbon based products like hydrocarbons and alcohols, see e.g. WO 2009/036095 A1 and WO 2010/044960 A1. DE 10 2010 019330 A1 discloses a method for producing lime in a shaft furnace including CO2 exhaust gas control, wherein the oxygen content of the combustion air may be increased by addition of technical oxygen. The document further discloses to supply exhaust gas to a bioreactor, wherein the resulting biomass may be used as fuel.

It has now surprisingly been found that the oxygen generated by photoautotrophic organisms is a cost effective source of oxygen for oxyfuel and other cement kilns for manufacturing cement. The same applies to lime kilns. The organisms can advantageously be fed with the CO₂ vented from the cement plant.

Thus, the present invention solves the problem of providing less costly oxygen for a process for operating a cement or lime plant comprising an oxyfuel kiln and/or oxyfuel calciner, wherein the oxygen is at least partly provided by photoautotrophic organisms. Furthermore, the oxygen generated by photoautotrophic organisms can also be used to provide oxygen enriched air for normal cement or lime kilns and calciners, allowing e.g. more alternative fuels to be used or a higher flame temperature. Therefore, the present invention also solves the problem of improving the combustion in a cement kiln and/ or calciner or in a lime kiln, wherein heat is generated by combustion of a fuel in the kiln and/or calciner and a gas fed to the kiln and/or to the calciner for combustion of the fuel contains an oxygen rich exhaust gas from a bioreactor containing photoautotrophic organisms. The problem of providing less costly oxygen and improving combustion is further solved with a cement or lime plant wherein the cement plant comprises a kiln and a calciner and the lime plant comprises a kiln, especially comprising an oxyfuel kiln and/or oxyfuel calciner, a bioreactor containing photoautotrophic organisms and means for passing oxygen generated by the organisms into the kiln and/or calciner. The problem is finally solved by using oxygen enriched exhaust gas from a bioreactor containing photoautotrophic organisms as full or partial replacement of air for combustion of a fuel in a kiln and/or calciner of a cement or lime plant for manufacturing cement or lime, respectively.

It is advantageous to use the exhaust gas from the cement or lime plant as feed gas for the photoautotrophic organisms in the bioreactor. This is especially beneficial for cement and lime plants operated in the oxyfuel mode, where the exhaust gas contains a high amount of CO₂. But it is also a good way of utilization for CO₂ exhausted by normal cement or lime plants. In the case of normal cement or lime plants an enrichment of CO₂ in the exhaust gas by any known means can be foreseen.

For cement and lime plants operated in the oxyfuel mode there exists a further problem. The cement or lime plant works continuously but the bioreactor only during day time, i.e. when sunlight is available. Thus, the problem of oxygen supply to the kiln (and/or calciner) during the night has to be solved. For the preferred utilization of CO₂ vented from the cement or lime plant as feed gas for the bioreactor the same problem arises for normal and oxyfuel operation, i.e. the bioreactor cannot consume CO₂ during the night. To solve this problem the generated oxygen and/or CO₂ is/are partly stored, so that the operating conditions of the kiln (and/or calciner) can be kept more or less constant during day and night. Advantageously, the gas(es) are stored by liquefying. While it seems possible to store all exhaust gas over the whole time it is expected to be more efficient to store only parts and use the rest directly as long as possible. Storage of parts needs more conduits and means for control and regulation, but it is expected to have a better energy efficiency.

It is of course also possible to supply oxygen from another source during the night, e.g. from water electrolysis that utilizes cheap surplus power in the electric supply network. Likewise, CO₂ vented during the night can be used in other ways than feeding it to the bioreactor. One possible use is conversion to carbon based products using the hydrogen generated by water electrolysis.

As a third alternative, especially for cement and lime plants that are not operated in the oxyfuel mode, the supply of extra oxygen can simply be stopped during the night and the fuel and/or conditions of burning are adapted accordingly.

It is especially preferred to apply the methods and plants according to the invention to the manufacturing of cement, specifically to making ordinary portland cement.

The invention will be illustrated further with reference to the attached figures, without restricting the scope to the specific embodiments described. If not otherwise specified any amount in % or parts is by volume and in the case of doubt referring to the total volume of the composition/mixture concerned. The invention further includes all combinations of described and especially of preferred featurest that do not exclude each other. A characterization as "approximately", "around" and similar expression in relation to a numerical value means that up to 10 % higher and lower values are included, preferably up to 5 % higher and lower values, and in any case at least up to 1 % higher and lower values, the exact value being the most preferred value or limit.

In the figures:
Figure 1 shows an oxyfuel cement kiln supplied with oxygen from a bioreactor,
Figure 2a and 2b show an oxyfuel kiln and bioreactor with storage of the gases,
Figure 3 shows a normal cement kiln supplied with oxygen from a bioreactor,
Figure 4a and 4b show a cement kiln with oxyfuel calciner,
Figure 5 shows a shaft lime kiln and
Figure 6 shows a rotary lime kiln.

In all figures the gas streams are shown with simple arrows and streams of solid material with wider arrows having a frame. Sunlight is depicted as wave. Inactive parts are shown in dotted lines.

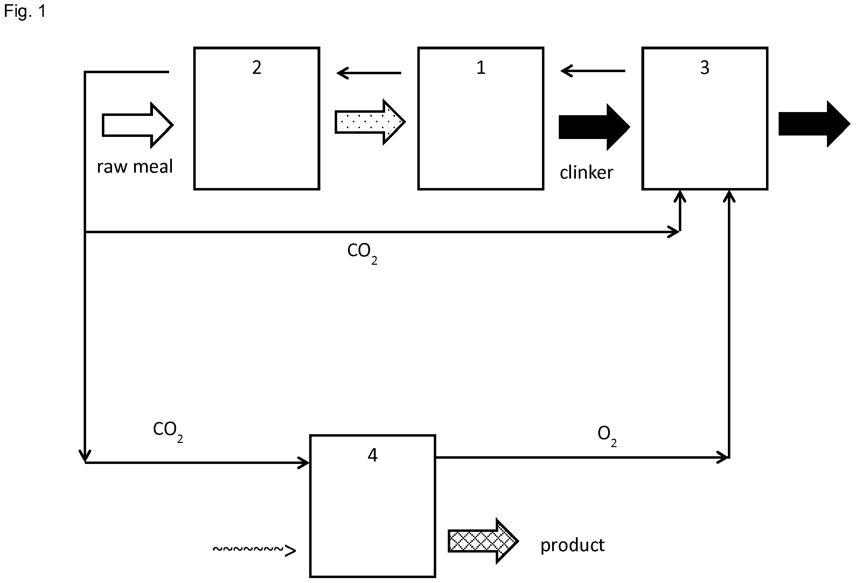

Figure 1 shows a cement plant operated in the oxyfuel mode comprising a cement kiln 1, a preheater or calciner 2 and a clinker cooler 3. The rest of the cement plant, like raw material feed for the kiln, corresponds to the known plants and is not shown or described, therefore. The bioreactor is designated 4.

The kiln 1 is operated in the oxyfuel mode, that means the gas entering the clinker cooler 3 does not contain any substantial amount of nitrogen, preferably it consists of oxygen and the recirculated gas containing unburnt oxygen and CO₂. It contains only traces of other gases like nitrogen, argon and other components of air, that would require an inadequate expenditure to be removed. The recirculated CO₂ is preferably dried. The gas entering the cooler 3 is preheated while the clinker cools. The preheated gas enters the kiln 1 where fuel is burnt to sinter the preheated or calcined raw meal to form the cement clinker which moves into the cooler 3. The hot gas is passed into the preheater/calciner 2, where the raw meal is preheated and usually also at least partly calcined. Typically, full calcination requires an additional burner.

The exhaust gas from the preheater/calciner 2 is recirculated to the cooler 3 and partly vented. Generally the vented gas contains 70 % or more CO₂ whereas the exhaust from a normal cement plant contains 20 % to 25 %.

In the shown embodiment, the vented gas is fed to the bioreactor 4 to be converted into valuable products by the organisms. In the bioreactor 4 photoautotrophic organisms convert CO₂ into valuable products using sunlight as energy source. Besides the valuable product the organisms generate oxygen, which is added to the gas entering the cooler 3 according to the invention. The oxygen could of course also be added to the kiln inlet.

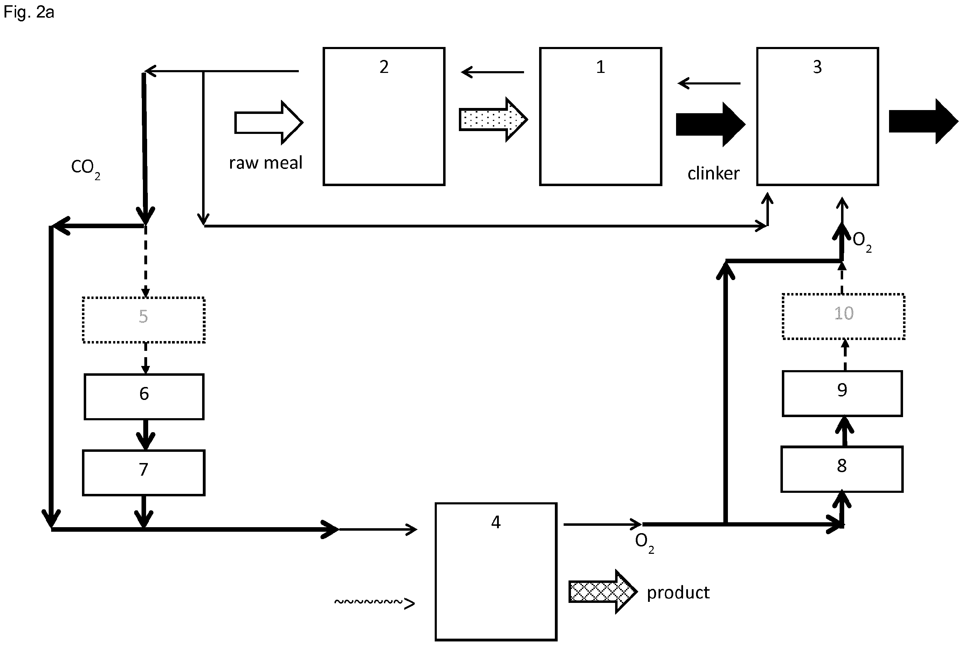

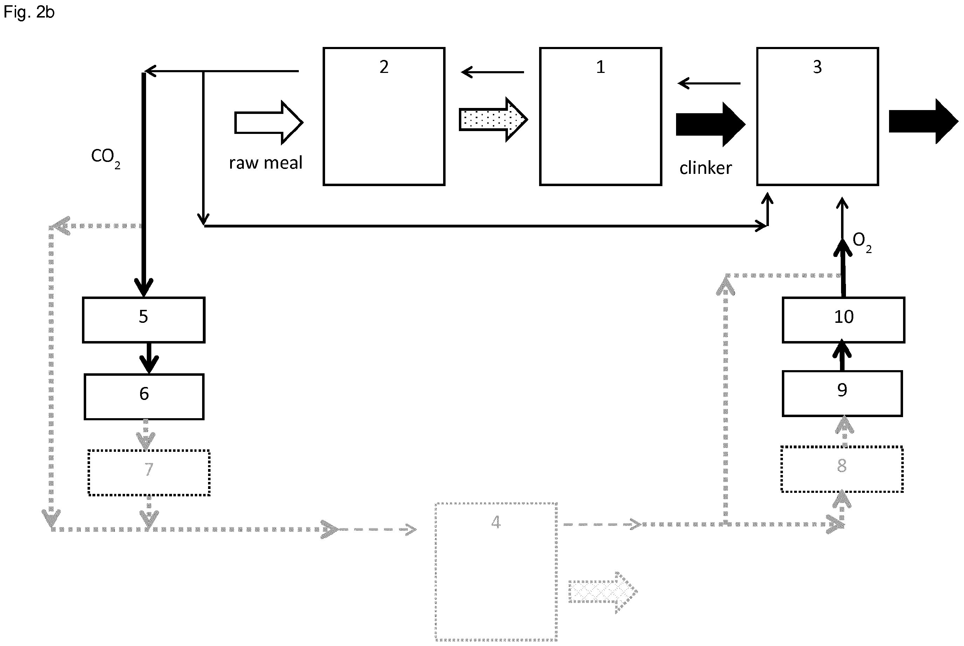

Figure 2a and 2b show a preferred embodiment of the invention. The parts which are identical to figure 1 have the same reference numbers and are not explained again. In addition to them, the device and process illustrated in figures 2a and 2b uses a storage of oxygen and gas vented from the cement plant, designated CO₂ in the following, to allow a 24 hours operation under similar conditions despite the lack of activity of the organisms in the bioreactor 4 during the night.

The day time operation is shown in figure 2a. Here the CO₂ vented from the cement plant is fed directly into the bioreactor 4. The bioreactor 4 provides oxygen. A part of the oxygen is liquefied in device 5 and stored in container 6, the remaining amount is fed into the cooler 3 (or kiln inlet). Vented gas liquefied in device 8 and stored in container 9 during the night is evaporated in evaporater 10.

Figure 2b shows the night time operation, where the bioreactor 4 does neither consume CO₂ nor generate oxygen. During the night the oxygen needed for the kiln 1 (and preheater/calciner 2) is obtained by evaporating oxygen stored in container 6 in the evaporater 7. The CO₂ vented from the cement plant is liquefied in device 8 and stored in container 9.

In all embodiments the process and device according to the invention can also be used when only the calciner is operated in the oxyfuel mode instead of the kiln and calciner. The CO₂ reduction achieved with only an oxyfuel calciner in comparison to a normal kiln will be less than for the oxyfuel kiln but still considerable. The advantage is that the necessary changes to the plant are less. Since oxyfuel operation requires to keep out air, the demands on the tightness of the cooler, kiln and preheater/calciner are high. Restricting these requirements to the preheater/calciner section will lower the demands noticeably.

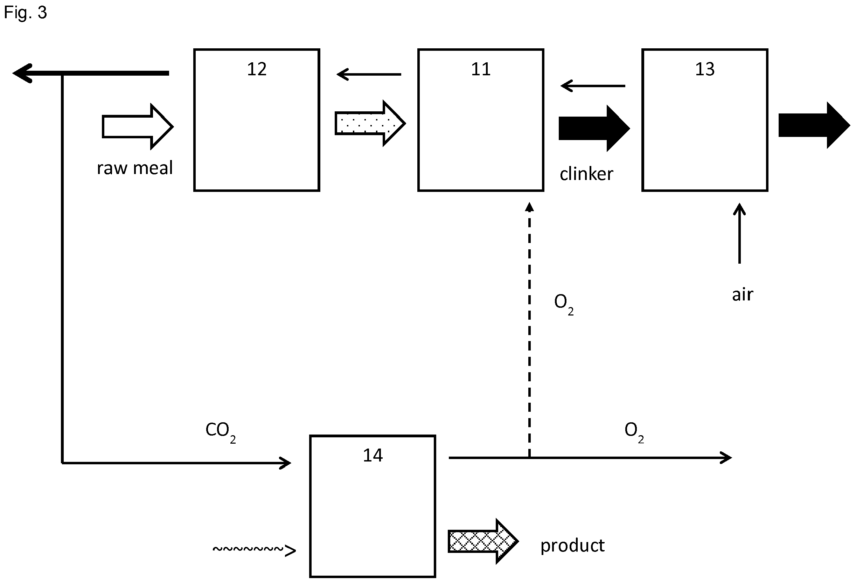

Figure 3 shows a normal cement plant, i.e. not operated in the oxyfuel mode. This cement plant comprises a kiln 11, a preheater/calciner 12 and a clinker cooler 13. The difference in view of the oxyfuel plant is that air and not oxygen is fed into the cooler 13 and that no gas is recycled to the cooler 13, all is vented. As shown by the dashed arrow, combustion in the kiln 1 can be improved by feeding oxygen from the bioreactor 14 to the clinker burner in addition to air. It is also advantageous here to feed the vented gas wich is enriched in CO₂ into the bioreactor 14. The bioreactor will operate efficiently with exhaust gas with a CO₂-concentration as low as 10%. However the exhaust gas of a regular operated kiln cannot be liquefied, and storage of the exhaust gas over night when it is gaseous would require unaffordable large storage facilities. Therefore, if full utilisation of the CO₂ is envisioned, it is required to reach > 70% CO₂, so that liquefying is possible and storage of the CO₂ as a liquid requires roughly 1000 times smaller storage facilities.

As noted previously, the bioreactor (4, 14) is only operating during day time while the cement plant runs 24 hours a day. Therefore, unless it is acceptable to operate the oxyfuel cement plant with differing conditions during night and day, some sort of oxygen supply has to be provided during the night. When no scheme as above described, i.e. a storage of part of the oxygen generated during the day, is possible or desired, oxygen supply as known in the art will be used during the night. The most common supply is by isolating oxygen from the ambient air, e.g. with the Linde process or some improved method.

Another possible approach is generation of oxygen by electrolysis of water, wherein it is desirable to utilize the hydrogen generated concurrently for converting the CO₂ to valuable products. Such a process is described e.g. in WO 2015/055349 A1.

With regard to the bioreactor (4, 14), the most preferred photoautotrophic organisms are ones of the type described in WO 2009/036095 A1 and WO 2010/044960 A1. These produce the valuable end product, for example ethanol, directly. However, is is also possible to rely on other known processes and organisms, like algea, that utilize light and CO₂ to build up biomass that is subsequently converted into the desired valuable end products.

For an exemplary oxyfuel kiln the mass balance for oxygen and carbon dioxide has been calculated and is shown in the following table 1.

**Table 1**

| | |
|---|---|
| clinker production | 125 tons/hour |
| oxygen needed (according to Technical Report TR ECRA 119/2012) | 35 tons/hour |
| CO₂ generated (865 kg/ton clinker) | 108 tons/hour |
| CO₂ uptake (assumed uptake efficiency of CO₂ 85 %) | 92 tons/hour |
| ratio of produced O₂ to CO₂ for ethanol producing cyano bacteria | 1,09 |
| ratio of produced O₂ to CO₂ for micro-algae | 1,7 |
| lower limit oxygen production | 100 tons/hour |

It can be seen, that for a typical cement kiln and a bioreactor dimensioned to be fed all the CO₂ evaporated from the kiln operated in the in oxyfuel mode at least 65 tons/hour oxygen are available for storage and/or other uses.

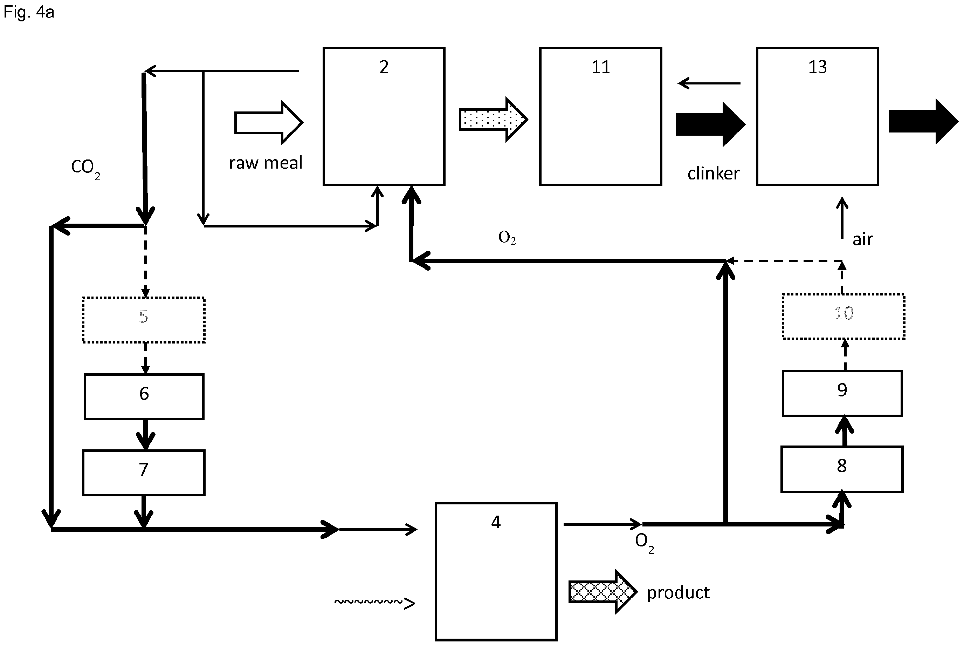

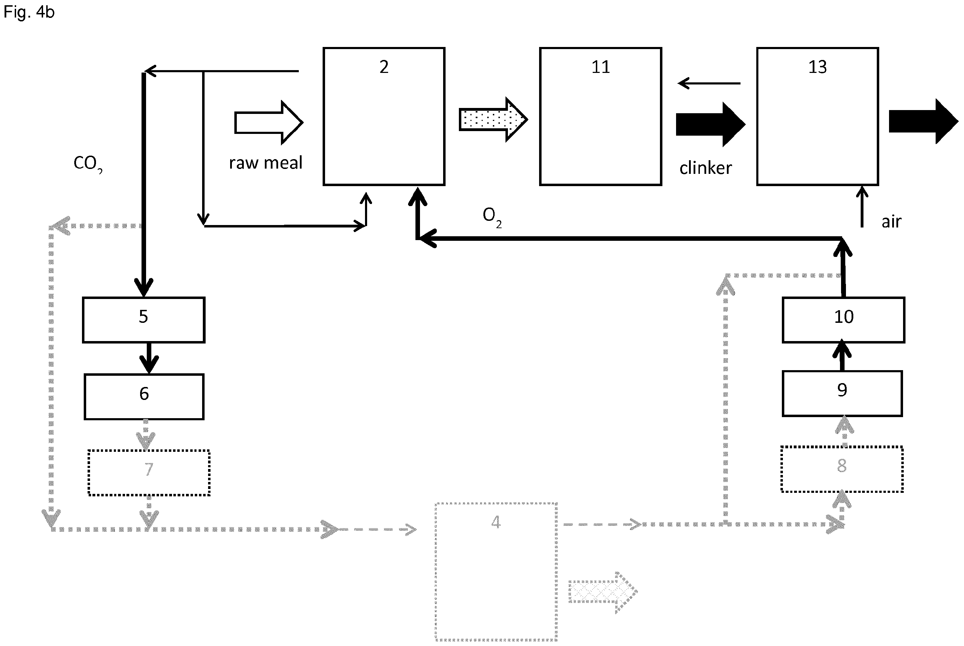

Figures 4a and 4b show a cement plant wherein only the calciner 2 is operated in the oxyfuel mode while the kiln 1 receives air for combustion, which is preheated in the cooler 3. The bioreactor 4 providing oxygen for the calciner 2 as well as the devices for oxygen and carbon dioxide liquefying, storage, and evaporation 5, 6, 7 and 8, 9, 10 are the same as in the embodiment shown in Figures 2a and 2b. Heated air from the cooler 3 and/or the kiln 1 can in the embodiment of figure 4a and 4b advantageously be passed into the raw materials and/or fuel drying.

The process according to the invention for operating a cement plant comprising a cement kiln and a calciner, wherein heat is generated by combustion of a fuel in the kiln and/or calciner, uses as gas for combustion of the fuel an oxygen rich exhaust gas from a bioreactor containing photoautotrophic organisms. Thereby, a cost effective source of oxygen is provided for improving combustion in the cement plant and utilizing the oxygen rich exhaust gas from the bioreactor generates an additional economical benefit for the reactor-plant.

In the preferred embodiment the cement plant is operated in the oxyfuel mode by using exhaust gas from the kiln and/or calciner together with the oxygen from the bioreactor as the gas fed to the kiln and/or calciner for combustion of the fuel. Thereby, a cost effective source of oxygen is provided for the oxyfuel cement plant and the gas vented from the cement plant is converted into valuable products by the organisms in the bioreactor.

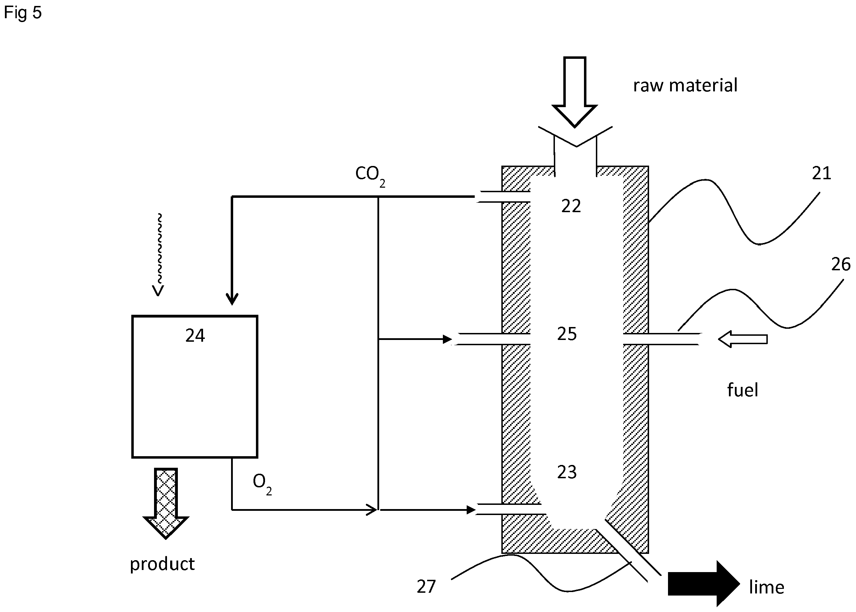

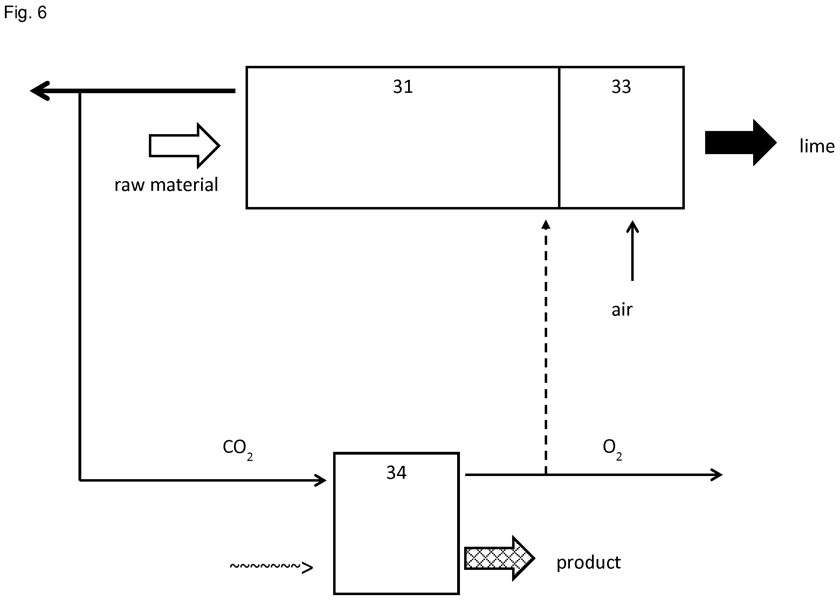

The illustrated devices and methods can be applied analogously to the manufacturing of lime as shown in figures 5 and 6.

Figure 5 shows a vertical shaft kiln 21 for lime production in the oxyfuel mode combined with a bioreactor 24. Calcium carbonate, e.g. limestone, is fed as raw material at the upper end into the kiln 21. The upper part of the kiln 21 forms a preheating zone 22 where the raw material is preheated by the hot gases flowing upwards, a typical temperature is about 1200 K. The exhaust gas, comprising carbon dioxide and remaining oxygen, is passed out of the kiln at the upper end and is partly recycled into the lowest part of the kiln 21, which forms the cooling zone 23. The carbon dioxide that has to be vented is fed to the bioreactor 24. The preheated raw material passes down the kiln 21 into the burning zone 25 which is located in the middle of the kiln 21 and has a typical temperature of about 1500 K. Typically fuel and oxygen are fed into the kiln 21 at the burning zone 25 via lances 26. The oxygen is provided by the bioreactor 24 and fed to the cooling zone 23 and/or the burning zone 25. The raw material is decomposed into lime and carbon dioxide in the burning zone 25 and the lime then passes downwards through the cooling zone 23 and is taken out at the product outlet 27. Recycled exhaust gas and/or added oxygen from the bioreactor are passed into the cooling zone 23 for cooling. The introduced gas is usually heated to a temperature of 600 to 750 K by the lime leaving the burning zone 25 at about 1200 K.

Figure 6 illustrates the manufacturing of lime in a rotary kiln 31. In this embodiment the kiln 31 is not operated in the oxyfuel mode and the oxygen produced by the bioreactor 34 may be used to improve the burning conditions in the kiln 31. The lime rotary kiln 31 has no separate preheater/calciner, the raw materials are fed directly into the kiln 31, so that preheating and burning (decomposition) both take place inside the kiln 31. The lime is cooled in cooler 33 by air and/or the oxygen from the bioreactor 34 that is passed into the kiln 31 in counterflow to the raw material. Exhaust gas from the kiln 31 is fed to the bioreactor 34, optionally after concentrating it in CO₂.

Of course, the lime plants may be equipped with the same oxygen and/or carbon dioxide storage devices as the cement plants described before. Also the same methods of providing oxygen and using carbon dioxide during the night can be applied.

### Reference numbers:

- 1: cement kiln
- 2: preheater/calciner
- 3: clinker cooler
- 4: bioreactor
- 5: oxygen liquefier
- 6: oxygen storage container
- 7: oxygen evaporater
- 8: CO₂ liquefier
- 9: CO₂ storage container
- 10: CO₂ evaporator

- 11: cement kiln
- 12: preheater/calciner
- 13: clinker cooler
- 14: bioreactor

- 21: lime shaft kiln
- 22: preheating zone
- 23: cooling zone
- 24: bioreactor
- 25: burning zone
- 26: lance for fuel and air/oxygen
- 27: product outlet

- 31: lime rotary kiln
- 33: cooler
- 34: bioreactor

## Claims

1. Process for operating a cement or lime plant comprising a cement or lime kiln (1, 11, 21, 31) and a calciner (2, 12) in case of a cement plant, wherein heat is generated by combustion of a fuel in the kiln (1, 11, 21, 31) and/or in the calciner (2, 12), wherein a gas fed to the kiln (1, 11, 21, 31) and the calciner (2, 12) or to the calciner (2, 12) for combustion of the fuel contains an oxygen rich exhaust gas from a bioreactor (4, 14, 24, 34) containing photoautotrophic organisms.

2. Process according to claim 1, wherein an exhaust gas from the kiln (1, 21) and/or calciner (2) is partly recycled into the kiln (1, 21) and/or calciner (2), operating the kiln (1, 21) and/or calciner (2) in the oxyfuel mode.

3. Process according to claim 2, wherein the gas fed to the the kiln (1, 21) and the calciner (2, 12) or to the calciner (2, 12) consists of the recycled exhaust gas from the kiln (1, 21) and/or calciner (2, 12) and the oxygen rich exhaust gas from the bioreactor (4, 14, 24).

4. Process according to claim 2 or 3, wherein the exhaust gas from the kiln (1, 11, 21, 31) and/or calciner (2, 12) that is not recycled into the kiln (1, 11, 21, 31) and/or calciner (2, 12) is passed into the bioreactor (4, 14, 24, 34) as feed gas for the photoautotrophic organisms.

5. Process according to anyone of claims 1 to 4, wherein the generated oxygen is partly stored during a time when the bioreactor (4, 24) generates oxygen and stored oxygen is fed to the kiln (1, 21) and/or calciner (2) during a time when the bioreactor (4, 24) does not generate oxygen.

6. Process according to anyone of claims 2 to 5, wherein the gas from the kiln (1, 21) and/or calciner (2) that is not recycled into the kiln (1, 21) and/or calciner (2), the vented gas, is stored during a time when the bioreactor (4, 24) does not generate oxygen and stored gas is fed to the bioreactor (4, 24) during a time when the bioreactor (4, 24) generates oxygen.

7. Process according to claim 1, wherein the exhaust gas from the kiln (11, 31) and/or calciner (12) is not recycled into the kiln (11, 31) or the calciner (12) and is passed into the bioreactor (14, 34) as feed gas for the photoautotrophic organisms.

8. Process according to claim 7, comprising the further step of enriching the exhaust gas in CO₂ prior to passing it into the bioreactor (14, 34).

9. Cement or lime plant comprising a cement or lime kiln (1, 11, 21, 31), in the case of a cement plant a calciner (2, 12), and at least one burner for generating heat by combustion of a fuel in the kiln (1, 11, 21, 31) and/or in the calciner (2, 12), **characterized in that** it further comprises a bioreactor (4, 14, 24, 34) containing photoautotrophic organisms and means for passing oxygen generated by the organisms into the kiln (1, 11, 21,31) and/or calciner (2, 12).

10. Cement or lime plant according to claim 9, wherein the cement or lime kiln (1, 11, 21, 31) and/or calciner (2, 12) is an oxyfuel kiln (1, 21) and/or oxyfuel calciner (2) receiving exhaust gas from the kiln (11, 21) and/or calciner (2) and the oxygen generated by the organisms as gas for the combustion of the fuel.

11. Cement or lime plant according to claim 10, further comprising devices for liquefying (5, 7) oxygen and exhaust gas from the kiln (1, 21) and/or calciner (2) that is not recycled into the kiln (1, 21) and/or calciner (2), the vented gas, containers (6, 8) for storing the liquefied oxygen and the vented gas, and devices for evaporating the stored oxygen and vented gas and feeding the oxygen into the kiln (1, 21) and/or calciner (2) and the vented gas into the bioreactor (4, 24), respectively.

12. Use of oxygen enriched exhaust gas from a bioreactor (4, 14, 24, 34) containing photoautotrophic organisms as full or partial replacement of air for combustion of a fuel in a kiln (1, 11) and/or a calciner (2, 12) of a cement plant or in a kiln (21, 31) of a lime plant.

## Patentansprüche

1. Verfahren zum Betreiben eines Zement- oder Kalkwerks, umfassend einen Zement- oder Kalkofen (1, 11, 21, 31) und einen Kalzinator (2, 12) im Falle eines Zementwerks, wobei Wärme durch Verbrennung eines Brennstoffs in dem Ofen (1, 11, 21, 31) und/oder in dem Kalzinator (2, 12) erzeugt wird, wobei ein Gas, das dem Ofen (1, 11, 21, 31) und dem Kalzinator (2, 12) oder dem Kalzinator (2, 12) zur Verbrennung des Brennstoffs zugeführt wird, ein sauerstoffreiches Abgas aus einem Bioreaktor (4, 14, 24, 34), der photoautotrophe Organismen enthält, enthält.

2. Verfahren gemäß Anspruch 1, wobei ein Abgas aus dem Ofen (1, 21) und/oder Kalzinator (2) teilweise in den Ofen (1, 21) und/oder Kalzinator (2) zurückgeführt wird, wobei der Ofen (1, 21) und/oder Kalzinator (2) im Oxyfuel-Modus betrieben wird.

3. Verfahren gemäß Anspruch 2, bei dem das dem Ofen (1, 21) und dem Kalzinator (2, 12) oder dem Kalzinator (2, 12) zugeführte Gas aus dem zurückgeführten Abgas des Ofens (1, 21) und/oder des Kalzinators (2, 12) und dem sauerstoffreichen Abgas aus dem Bioreaktor (4, 14, 24) besteht.

4. Verfahren gemäß Anspruch 2 oder 3, bei dem das Abgas aus dem Ofen (1, 11, 21, 31) und/oder Kalzinator (2, 12), das nicht in den Ofen (1, 11, 21, 31) und/oder Kalzinator (2, 12) zurückgeführt wird, als Einspeisegas für die photoautotrophen Organismen in den Bioreaktor (4, 14, 24, 34) geleitet wird.

5. Verfahren gemäß einem der Ansprüche 1 bis 4, wobei der erzeugte Sauerstoff während einer Zeit, in der der Bioreaktor (4, 24) Sauerstoff erzeugt, teilweise gespeichert wird und der gespeicherte Sauerstoff während einer Zeit, in der der Bioreaktor (4, 24) keinen Sauerstoff erzeugt, dem Ofen (1, 21) und/oder Kalzinator (2) zugeführt wird.

6. Verfahren gemäß einem der Ansprüche 2 bis 5, wobei das Gas aus dem Ofen (1, 21) und/oder Kalzinator (2), das nicht in den Ofen (1, 21) und/oder Kalzinator (2) zurückgeführt wird, das entlüftete Gas, während einer Zeit, in der der Bioreaktor (4, 24) keinen Sauerstoff erzeugt, gespeichert wird und gespeichertes Gas während einer Zeit, in der der Bioreaktor (4, 24) Sauerstoff erzeugt, dem Bioreaktor (4, 24) zugeführt wird.

7. Verfahren gemäß Anspruch 1, wobei das Abgas aus dem Ofen (11, 31) und/oder Kalzinator (12) nicht in den Ofen (11, 31) oder den Kalzinator (12) zurückgeführt wird und als Einspeisegas für die photoautotrophen Organismen in den Bioreaktor (14 34) geleitet wird.

8. Verfahren gemäß Anspruch 7, umfassend den weiteren Schritt des Anreicherns des Abgases an CO₂, bevor es in den Bioreaktor (14, 34) geleitet wird.

9. Zement- oder Kalkwerk mit einem Zement- oder Kalkofen (1, 11, 21, 31), im Falle einer Zementanlage einem Kalzinator (2, 12) und mindestens einem Brenner zur Erzeugung von Wärme durch Verbrennung eines Brennstoffes in dem Ofen (1, 11, 21, 31) und/oder in dem Kalzinator (2, 12), **dadurch gekennzeichnet, dass** sie außerdem einen Bioreaktor (4, 14, 24, 34) umfasst, der photoautotrophe Organismen und Mittel zum Leiten von Sauerstoff, der von den Organismen erzeugt wird, in den Ofen (1, 11, 21, 31) und/oder Kalzinator (2, 12) umfasst.

10. Zement- oder Kalkwerk gemäß Anspruch 9, wobei der Zement- oder Kalkofen (1, 11, 21, 31) und/oder Kalzinator (2, 12) ein Oxyfuel-Ofen (1, 21) und/oder Oxyfuel- Kalzinator (2) ist, der Abgas aus dem Ofen (11, 21) und/oder Kalzinator (2) und den von den Organismen erzeugten Sauerstoff als Gas zur Verbrennung des Brennstoffs empfängt.

11. Zement- oder Kalkwerk gemäß Anspruch 10, weiterhin umfassend Vorrichtungen zum Verflüssigen (5, 7) von Sauerstoff und Abgas aus dem Ofen (1, 21) und/oder Kalzinator (2), das nicht in den Ofen (1, 21) und/oder Kalzinator (2) zurückgeführt wird, das entlüftete Gas, Behälter (6, 8) zum Speichern des verflüssigten Sauerstoffs und des entlüfteten Gases und Vorrichtungen zum Verdampfen des gespeicherten Sauerstoffs und entlüfteten Gases und zum Zuführen des Sauerstoffs in den Ofen (1, 21) und/oder Kalzinator (2) und des entlüfteten Gases in den Bioreaktor (4, 24).

12. Verwendung von mit Sauerstoff angereichertem Abgas aus einem Bioreaktor (4, 14, 24, 34) enthaltend photoautotrophe Organismen als vollständigen oder teilweisen Ersatz von Luft zur Verbrennung eines Brennstoffs in einem Ofen (1, 11) und/oder einem Kalzinator (2, 12) eines Zementwerks oder in einem Ofen (21, 31) eines Kalkwerks.

## Revendications

1. Procédé d'exploitation d'une usine de ciment ou de chaux comprenant un four à ciment ou à chaux (1, 11, 21, 31) et un four de calcination (2, 12) dans le cas d'une usine de ciment, dans lequel de la chaleur est générée par combustion d'un carburant dans le four (1, 11, 21, 31) et/ou dans le four de calcination (2, 12), dans lequel un gaz alimenté vers le four (1, 11, 21, 31) et le four de calcination (2, 12) ou vers le four de calcination (2, 12) en vue d'une combustion du carburant contient un gaz d'échappement riche en oxygène issu d'un bioréacteur (4, 14, 24, 34) contenant des organismes photoautotrophes.

2. Procédé selon la revendication 1, dans lequel un gaz d'échappement issu du four (1, 21) et/ou du four de calcination (2) est partiellement recyclé dans le four (1, 21) et/ou le four de calcination (2), en exploitant le four (1, 21) et/ou le four de calcination (2) dans le mode oxycombustion.

3. Procédé selon la revendication 2, dans lequel le gaz alimenté vers le four (1, 21) et le four de calcination (2, 12) ou vers le four de calcination (2, 12) est constitué du gaz d'échappement recyclé issu du four (1, 21) et/ou du four de calcination (2, 12) et du gaz d'échappement riche en oxygène issu du bioréacteur (4, 14, 24).

4. Procédé selon la revendication 2 ou 3, dans lequel le gaz d'échappement issu du four (1, 11, 21, 31) et/ou du four de calcination (2, 12) qui n'est pas recyclé dans le four (1, 11, 21, 31) et/ou le four de calcination (2, 12) est entraîné dans ledit réacteur (4, 14, 24, 34) en tant que gaz d'alimentation pour les organismes photoautotrophes.

5. Procédé selon l'une quelconque des revendications 1 à 4, dans lequel l'oxygène généré est partiellement stocké pendant une durée pendant laquelle le bioréacteur (4, 24) génère de l'oxygène et l'oxygène stocké est alimenté vers le four (1, 21) et/ou le four de calcination (2) pendant une durée pendant laquelle le bioréacteur (4, 24) ne génère pas d'oxygène.

6. Procédé selon l'une quelconque des revendications 2 à 5, dans lequel le gaz issu du four (1, 21) et/ou du four de calcination (2) qui n'est pas recyclé dans le four (1, 21) et/ou le four de calcination (2), à savoir le gaz d'évent, est stocké pendant une durée pendant laquelle le bioréacteur (4, 24) ne génère pas d'oxygène et le gaz stocké est alimenté vers le bioréacteur (4, 24) pendant une durée pendant laquelle le bioréacteur (4, 24) génère de l'oxygène.

7. Procédé selon la revendication 1, dans lequel le gaz d'échappement issu du four (11, 31) et/ou du four de calcination (12) n'est pas recyclé dans le four (11, 31) ou le four de calcination (12) et est entrainé dans le bioréacteur (14, 34) en tant que gaz d'alimentation pour les organismes photoautotrophes.

8. Procédé selon la revendication 7, comprenant l'étape supplémentaire consistant à enrichir le gaz d'échappement en CO₂ préalablement à son entrainement dans le bioréacteur (14, 34).

9. Usine de ciment ou de chaux comprenant un four à ciment ou à chaux (1, 11, 21, 31), à savoir un four de calcination (2, 12) dans le cas d'une usine de ciment, et au moins un brûleur permettant de générer de la chaleur par combustion d'un carburant dans le four (1, 11, 21, 31) et/ou dans le four de calcination (2, 12), **caractérisée en ce qu'**elle comprend en outre un bioréacteur (4, 14, 24, 34) contenant des organismes photoautotrophes et un moyen permettant d'entrainer de l'oxygène généré par les organismes dans le four (1, 11, 21, 31) et/ou le four de calcination (2, 12).

10. Usine de ciment ou de chaux selon la revendication 9, dans laquelle le four à ciment ou à chaux (1, 11, 21, 31) et/ou le four de calcination (2, 12) est un four à oxycombustion (1, 21) et/ou un four de calcination à oxycombustion (2) recevant du gaz d'échappement en provenance du four (11, 21) et/ou du four de calcination (2) et l'oxygène généré par les organismes en tant que gaz destiné à la combustion du carburant.

11. Usine de ciment ou de chaux selon la revendication 10, comprenant en outre des dispositifs permettant de liquéfier (5, 7) de l'oxygène et du gaz d'échappement en provenance du four (1, 21) et/ou du four de calcination (2) qui n'est pas recyclé dans le four (1, 21) et/ou le four de calcination (2), à savoir le gaz d'évent, des conteneurs (6, 8) permettant de stocker l'oxygène liquéfié et le gaz d'évent, et des dispositifs permettant d'évaporer l'oxygène et le gaz d'évent stockés et d'alimenter l'oxygène vers le four (1, 21) et/ou le four de calcination (2) et le gaz d'évent vers le bioréacteur (4, 24), respectivement.

12. Utilisation de gaz d'échappement enrichi en oxygène en provenance d'un bioréacteur (4, 14, 24, 34) contenant des organismes photoautotrophes en tant que remplacement complet ou partiel de l'air pour la combustion d'un carburant dans un four (1, 11) et/ou un four de calcination (2, 12) d'une usine de ciment ou dans un four (21, 31) d'une usine de chaux.
